(19)

Europäisches Patentamt

European Patent Office

Office européen des brevets

(11)  **EP 3 144 005 A1**

(12)  **EUROPEAN PATENT APPLICATION**
published in accordance with Art. 153(4) EPC

(43) Date of publication:
**22.03.2017  Bulletin 2017/12**

(21) Application number: **15792247.7**

(22) Date of filing: **08.04.2015**

(51) Int Cl.:
*A61K 36/74* (2006.01)     *A61K 36/43* (2006.01)
*A61K 36/8962* (2006.01)     *A61P 15/00* (2006.01)

(86) International application number:
**PCT/KR2015/003506**

(87) International publication number:
**WO 2015/174636 (19.11.2015 Gazette 2015/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **13.05.2014  KR 20140057386**

(71) Applicant: **Industrial Cooperation Foundation
Chonbuk National University
Jeonju-si, Jeollabuk-do 561-756 (KR)**

(72) Inventor: **PARK, Jong Kwan
Jeonju-si
Jeollabuk-do 560-735 (KR)**

(74) Representative: **Grünecker Patent- und
Rechtsanwälte
PartG mbB
Leopoldstraße 4
80802 München (DE)**

(54)  **COMPOSITION FOR PREVENTING AND TREATING MALE INFERTILITY, CONTAINING MIXED HERBAL EXTRACT AS ACTIVE INGREDIENT AND USE THEREOF**

(57)     The present invention relates to a composition and health functional food for preventing and treating male infertility using a mixed herbal mixture. Through an *in vitro* sperm mobility promotion effect evaluation method experiment for investigating effects of an herbal extract of *Morinda officinalis, cuscuta seed,* and/or *Allium cepa* of the present invention on changes in sperm count and mobility in human semen, it was verified that the samples of the present invention increased sperm mobility. In addition, through an *in vivo* varicocele animal model evaluation method for investigating effects of the herbal extract on changes in sperm count and mobility in an *in vino* varicocele animal model, it was verified that the samples of the present invention increased the sperm count in the semen obtained from spermiducts of a sample-administered group among varicocele-induced groups. Therefore, the herbal extract of the present invention can be usefully used in a pharmaceutical composition, health functional food, and health supplement food for preventing and treating male infertility.

EP 3 144 005 A1

**Description**

[Detailed Description of the invention]

[Technical Field]

The present invention is related to a composition comprising the combined herb extract for treating and preventing male infertility, specifically, a composition comprising the combined herb extract of *Morinda Officinalis root, Cuscuta seed,* and *Allium cepa bulb* for the prevention and treatment of male infertility and the use thereof.

There has been reported that varicocele, the most treatable cause of male factor infertility, is characterized by the tortuosity and dilation of the veins of the pampiniform plexus within the spermatic cord and the prevalence of varicocele is 19% to 41% in men with primary infertility with male and more than 80% in men with secondary infertility (Agarwal A, Deppinder F, Cocuzza M. Agarwal R. Short RA, Sabanegh E. et al, (2007) Efficacy of varicocelectomy in improving semen parameters: new meta-analytical approach. Urology 70, pp532-538*;* Agarwal A, Sharma RK, Desai NR, Prabakaran S, Tabanegh E. (2009) Role of Oxidative Stress is Pathogenesis of Varicocele and Infertility, Urology 73, pp461-469).

Most frequently performed surgical procedure for the treatment of male infertility caused by varicocele, is varicocelectomy including non-microsurgical approach method, laparoscopic or microsurgical varicocelectomy, which have been performed from long years ago (Mehta A & Goldstein M. (2012) Microsurgical varicocelectomy: a review. Asian J. Androl., 15, pp56-60). Although surgical correction of varicocele in infertile men has demonstrated improved parameters in 50 - 80% of patients performed by varicocelectomy (pregnancy rates, 31 - 74%), (Cheng D. Zheng XM, Li SW, Yang ZW & Hu LQ. (2006) Effects of epidermal growth factor on sperm content and motility of rats with surgically induced varicoceles. Asian J Androl 8, 713-717. Ding H, Tian JQ, Du W, Zhang LY, Wang HZ & Wang ZP. (2012) Open non-microsurgical, laparoscopic or open microsurgical varicocelectomy for male infertility: a meta-analysis of randomized controlled trials. BJU Int 110. 1536-1542; Fisch H & Hyun G. (2012) Varicocele repair for low testosterone. Curr Opin Urol 22, 495-498; Fretz PC & Sandlow JI. (2002) Varicocele: current concepts in pathophysiology, diagnosis, and treatment. Urol Clin North Am 29. 921-937; Mehta A & Goldstein M. (2013) Microsurgical varicocelectomy: a review. Asian J Androl 15. 56-60; Tanriverdi O, Miroglu C, Horasanli K. Altay B, Caliskan KC & Gumus E. (2006) Testicular blood flow measurements and mean resistive index values after microsurgical and high ligation varicocelectomy. Urology 67. 1262-1265), some reports have issued that varicocele repair does not seem to be an effective treatment for male or unexplained subfertility such as increased pregnancy failure rate of fertile postoperation etc (Evers JL & Collins JA. (2003) Assessment of efficacy of varicocele repair for male subfertility: a systematic review. The Lancet 361, 1849-1852; Ficarra V. Cerruto MA. Liguori G, Mazzoni G, Minucci S, Tracia A, et al. (2006) Treatment of varicocele in subfertile men: the Cochrane review a contrary opinion. Eur Urol 49, 258-263).

Although the exact mechanism by which the impaired testicular function in patients with varicocele causes to infertility, has not fully elucidated yet till now, some of hypothetical mechanisms have been suggested to study various varicocele related testicular dysfunctions including blood flow alteration in the testis, scrotal temperature elevations, abnormal hormone values, excessive reactive oxygen species (ROS) and so on (Agarwal A, Sharma RK, Desai NR. Prabakaran S, Tavares A & Sabanegh E. (2009) Role of Oxidative Stress in Pathogenesis of Varicocele and Infertility. Urology 73, 461-469; Fretz PC & Sandlow JI. (2002) Varicocele: current concepts in pathophysiology, diagnosis, and treatment. Urol Clin North Am 29, 921-937).

ROS (Reactive oxygen species) acting as a modulator of vital physiological intracellular processes is one of by-product reproduced from oxygen metabolism and energy production process. Small amount of ROS plays important role in controlling the spermatozoa function in male reproductive tract. Under abnormal condition, the oxidative stress follows where the increase of ROS level or reduction of anti-oxidative ability occurs. Specifically, oxidative stress causes to the modified level of expressed protein in the sperm, resulting in the deficiency of molecule and gene (Sharma R, Agarwal A, Mohanty G. Hamada AJ, Gopalan B, Willard B, et al. (2013) Proteomic analysis of human spermatozoa proteins with oxidative stress. Reprod Biol Endocrinol 11, 48).

Normally, the pregnancy rate of healthy couple reaches to 20-25% at the 1st month, 75% at the half year, and 85-90% at one year after the sexual relation. In clinical, the ratio of infertile patients among the married couples is presumed to become about 13 - 20% in the married couples (about 1,400,000 couples) in South Korea under the condition that the "infertility" is defined by the failed cases to pregnancy one year after the sexual relation. Among them, 35-50% infertility is caused by the health problem of men and about 30% of the problem results from the reduced motility of spermatozoa. Male infertility is one of the important and severe diseases of the outpatients, which becomes to 4% of the number of outpatients in urological hospital.

Recently, various ART (assisted reproductive technique) such as IVF (in vitro fertilization), micromanipulation technique etc have been developed due to the advanced reproductive biology or ART. However, the techniques have also some disadvantages and problems, for example, low pregnancy rate of only 20 to 40%, need for excessive expense, difficulty in applying on woman because of pain complaint, occurrence of complication and so on.

External fertilization also has the problem such as gene contamination, excessive expense etc.

Accordingly, the most safe and ideal target to increase the pregnancy rate of married couple, has been focused to develop the effective therapy for increasing the spontaneous pregnancy rate in the field of urological area and gynecological area till now.

In spite of the developed therapy of infertility, the effective treating agent to treat a male infertility and improving the sperm motility has not yet developed till now and there has been few studies on the drug which can improve the pregnancy rate. One of the current approaches to improve the semen parameters of men with low sperm motility, L-carnitine has been developed as a treating agent however the drug have been reported to show little apparent efficacy (Mark sigman, Stacy Glass. Janice Campagnone, John L. Reyor, Carnitine for the treatment of idiopathic asthenospermia: a randomized. double-blind, placebo-controlled trial. Fertility and Sterility Vol 85, No 5, 2006)

*Morinda Officinalis root,* a root of *Morinda Officinalis* belongs to Rubiaceae family and distributed at Southern region of China has been reported to comprise vitamin C, saccharide etc (B. S. Chung et al., YoungRim Press, 2nd Edition, DohaeHyangYakDaeSaJeon., pp927-928, 1998).

*Cuscuta seed,* a seed of *Cuscuta japonica,* or the like belongs to Convolvulaceae family has been reported to comprise resin glycoside, saccharide, vitamin A, beta-carotene, gamma-carotene, teraxanthin, lutein etc (B. S. Chung et al., YoungRim Press, 2nd Edition, DohaeHyangYak DaeSaJeon, pp882-883, 1998).

*Allium cepa bulb,* a bulb of *Allium cepa* belongs to Liliaceae family which is originated in Siberia or Altai has been reported to comprise thiol, dimethyl disulfide, diallyl disulfide, caffeic acid, ferulic acid, sinapic acid etc (B. S. Chung et al., YoungRim Press, 2nd Edition, DohaeHyangYakDaeSaJeon., pp155-156, 1998).

However, there has been not reported or disclosed about the therapeutic effect of the extract of combined herbs consisting *Morinda officinalis root, Cuscuta seed* and *Allium cepa bulb* as described above on male infertility disease in any of above cited literatures, the disclosures of which are incorporated herein by reference.

Therefore, the present inventors have endeavored to find the effective herb formulation for treating and preventing male infertility disease and the combined herb composition shows potent treating effect on human infertility, which is confirmed by various experiments, for example, *In vitro* sperm quality promoting effect evaluation as well as *in vivo* varicocele animal model evaluation method for investigating effects of the herbal extract on changes in sperm count and mobility in an in vivo varicocele animal model. Finally, the present inventors have found that the above-described combined herb extract is effective in treating and preventing male infertility disease.

[Technical Solution]

The technical solution to solve the problem of the background art is for the development of novel approach to increase the pregnancy rate through the promotion of sperm motility and then to recover the previous high pregnancy rate in the end.

According to one aspect, the present invention provides a pharmaceutical composition comprising the combined herb extract of *Morinda Officinalis root, Cuscuta seed,* and *Allium cepa bulb,* as an active ingredient for preventing and treating a male infertility disease.

The present invention also provides a health functional food comprising the combined herb extract of *Morinda Officinalis root, Cuscuta seed,* and *Allium cepa bulb* for the prevention or improvement of male infertility disease as an active ingredient to prevent and improve a male infertility disease.

The extract disclosed herein comprises the combined herb extract, i.e., *Morinda Officinalis root, Cuscuta seed,* and *Allium cepa bulb* with the mixed ratio based on the dried weight of each herb (w/w) ranging from 0.01 -100: 0.01-1: 1-100(w/w), preferably, 0.1-50: 0.1-1: 1-50(w/w), more preferably, 1 - 30: 1: 1-30 (w/w) in the present invention.

Specifically, the term "extract" disclosed herein comprises the extract soluble in distilled water, $C_1$-$C_4$ alcohols or the mixtures thereof, preferably, water, ethanol or the mixture thereof.

Specifically, the term "male infertility disease" disclosed herein comprises all the male infertility disease, for example, not intended to limit thereto, a male infertility disease caused by the adverse response of anti-cancer agent such as cisplatin, vinblastine, bleomycin and the like; azoospermia (sperm count: none), oligospermia (sperm count: $\leq$2 x$10^7$/ml), asthenospermia (ratio of high motile sperm: $\leq$40%), teratospermia (ratio of normal sperm: $\leq$40%) and the like (*See*, Korean Society for sexual medicine and andrology, Textbook of andrology, 2010).

An inventive herb composition may be prepared in accordance with the following preferred embodiment.

For example, the present invention also provides a method for preparing the inventive herb extract comprising the steps of; washing dried *Morinda Officinalis root, Cuscuta seed,* or *Allium cepa bulb,* respectively and adding 1 to 20-fold, preferably, 1 to 10-fold volume of distilled water, alcohols such as methanol, ethanol, and the like, or the mixtures thereof, preferably, distilled water, ethanol or the mixture thereof, thereto at 1st step; extracting each solution with the extraction method by the extraction with hot water, cold water, or ultra-sonication extraction, preferably, hot water extraction at the temperature ranging from 50°C ~ 120°C, preferably, about 80°C ~ 100°C, for the period ranging from 1 to 5 hours, preferably, 2 to 4 hours at 2$^{nd}$ step; repeating the above-described extraction process to collect each filtrate with filtration, drying through freeze drying, natural air drying or hot air drying process, preferably freeze drying process to obtain respective dried extract of *Morinda Officinalis root, Cuscuta seed,* or *Allium cepa bulb* at 3$^{rd}$ step; and mixing the respective dried extract of each herb(*Morinda Officinalis root: Cuscuta seed: Allium cepa bulb*) with the mixed ratio based on the dried weight of each herb (w/w) ranging from 0.01 -100: 0.01-1: 1-100(w/w), preferably, 0.1-50: 0.1-1: 1-50(w/w), more preferably, 1 - 30: 1: 1-30 (w/w) to prepare inventive extract of the present invention.

It is another object of the present invention to provide a process for preparing the extract of the present invention as described above for the preparation of composition effective in treating or preventing the purposed diseases.

It is still another object of the present invention to provide a pharmaceutical composition or health functional food comprising the herb extract of the above-mentioned herb obtained by the above described process as an active ingredient for preventing and treating male infertility disease.

The inventive composition of the present invention shows potent treating effect on human infertility, which is confirmed by various experiments, for example, *In vitro* sperm quality promoting effect evaluation as well as *in vivo* varicocele animal model evaluation method for investigating effects of the herbal extract on changes in sperm count and mobility in an in vivo varicocele animal model, it was verified that the inventive combined extract showed more potent increasing effect on the sperm count and sperm motility. Therefore, the herbal extract of the present invention can be usefully used in a pharmaceutical composition, health functional food, and health supplement food for preventing and treating male infertility.

The pharmaceutical composition for treating purposed diseases could contain about 0.01 to 99 w/w% the above herb extract of the present invention based on the total weight of the composition.

However, the amount and each component of the above-mentioned composition can be varied with the patient's condition, development of patient's disease, the sort of disease etc.

The inventive composition may additionally comprise conventional carrier, adjuvants or diluents in accordance with a using method.

The herb composition according to the present invention can be formulated in oral dosage form such as powder, granule, tablet, capsule, suspension, emulsion, syrup, aerosol and the like; topical preparation; or injection solution. The herb composition according to the present invention can be provided as a pharmaceutical composition containing pharmaceutically acceptable carriers, adjuvants or diluents, e.g., lactose, dextrose, sucrose, sorbitol, mannitol, xylitol, erythritol, maltitol, starches, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, magnesium stearate and mineral oil. The formulations may additionally include excipients such as fillers, bulking agents, binders, wetting agents, disintegrating agents, surfactants, diluents and the like. The solid oral dosage form comprises tablet, pill, powder, granule, capsule and the like and the solid oral dosage form is prepared by adding at least one excipient such as starch, calcium carbonate, sucrose, lactose or gelatin and the like to the herb extract. Lubricant such as magnesium stearate or talc may be used. The aqueous oral dosage form comprises suspension, oral solution, emulsion, syrup and the aqueous oral dosage form may comprise several excipients such as wetting agents, sweetener flavoring agents, preservatives, as well as water, liquid paraffin. The parenteral dosage form comprises sterilized aqueous solution, nonaqueous solvent, suspension, emulsion, lyophilized preparation, suppository, and the like. Suitable examples of the carriers include propylene glycol, polyethylene glycol, vegetable oils such as olive oil, injectable ester such as ethyl oleate. Base for suppository may include witepsol, macrogol, tween 61, cacao butter, laurin, glycerogelatine etc., but are not limited to them.

The desirable dose of the inventive composition varies depending on the condition and the weight of the subject, severity, drug form, route and period of administration, and may be chosen by those skilled in the art. However, in order to obtain desirable effects, it is generally recommended to administer at the amount ranging 0.01mg/kg to 10g/kg, preferably, 1mg/kg to 1g/kg by weight/day of the inventive composition of the present invention. The dose may be administered in a single or multiple doses per day.

The pharmaceutical composition of present invention can be administered to a subject animal such as mammals (rat, mouse, domestic animals or human) *via* various routes. All modes of administration are contemplated, for example, administration can be made orally, rectally or by intravenousinjection.

It is still another object of the present invention to provide a sperm motility improving agent comprising the combined herb extract of *Morinda Officinalis root*, *Cuscuta seed,* and *Allium cepa bulb,* as an active ingredient for preventing and treating male infertility disease.

It is still the other object of the present invention to provide a treating agent for male infertility by way of improving sperm motility comprising the combined herb extract of *Morinda Officinalis root, Cuscuta seed,* and *Allium cepa bulb* as an active ingredient.

It is still the other object of the present invention to provide a sperm storing additive for IVF (in vitro fertilization) comprising the combined herb extract of *Morinda Officinalis root, Cuscuta seed,* and *Allium cepa bulb* for improving fertilization rate of IVF.

It is the other object of the present invention to provide a method of treatment comprising administering of the composition comprising the combined herb extract of *Morinda Officinalis root, Cuscuta seed,* and *Allium cepa bulb* to a subject in need of treatment or prevention of male infertility disease.

It is another object of the present invention to provide a use of the combined herb extract of *Morinda Officinalis root, Cuscuta seed* and *Allium cepa bulb* as an active ingredient for manufacture of medicament employed for treating or preventing male infertility disease in human or mammal.

In accordance with one aspect of the present invention, there provided a health functional food comprising the combined herb extract of *Morinda Officinalis root, Cuscuta seed,* and *Allium cepa bulb* for the prevention or improvement of male infertility disease as an active ingredient.

The term "a health functional food" defined herein" comprises the functional food having enhanced functionality such as physical functionality or physiological functionality by adding the extract of the present invention to conventional food to prevent or improve the purposed diseases in human or mammal and stipulated by the Law for Health Functional Foods 6727 in Republic of Korea.

The health functional food composition for preventing and improving purposed diseases could contain about 0.01 to 95 w/w%, preferably 1 to 80 w/w% of the above herb composition of present invention based on the total weight of the composition.

Moreover, the inventive extract of the present invention also can be used as a main component or additive and aiding agent in the preparation of various functional health food and health supplement food for the prevention or improvement of male infertility disease or the improvement of testicular function.

The inventive health functional food may be prepared and processed by the form of pharmaceutically acceptable dosage form such as powder, granule, tablet, capsule, pills, suspension, emulsion, syrup and the like; or the functional health food form such as tea bag, leached tea, health beverage type and the like.

It is the other object of the present invention to provide a health supplement food comprising the combined herb extract of *Morinda Officinalis root, Cuscuta seed,* and *Allium cepa bulb, as a main component,* for the prevention or improvement of male infertility diseases.

The above-mentioned term "as a main component" means that the above health supplement food comprises about 30 to 99 (w/w%), preferably 50 to 99 (w/w%), more preferably 70 to 99 (w/w%) of the inventive extract of present invention based on the total weight of the composition.

When the combined herb extract of the present invention is used as a component in the health functional beverage composition, the health functional beverage composition can comprise other component such as flavoring agent or natural carbohydrate without limits like that typical beverage composition. Examples of the natural carbohydrate comprise monosaccharide such as glucose, fructose etc; disaccharide such as maltose, sucrose etc; and polysaccharide, for example, sugar such as dextrin, cyclodextrin, and sugar alcohol such as xylitol, sorbitol, erythritol. Natural flavoring agent (thaumatin, stevia extract (rebaudioside A, glycyrrhizin, etc)) and synthetic flavoring agent (saccharin, aspartame, etc) may be added in the health functional beverage composition. The amount of natural carbohydrate generally ranges from about 1 to 20g, preferably about 5 to 12g per 100ml of the present composition.

When the combined herb extract of the present invention is used as a food additive of the health food, the combined herb extract may be added intact or used with other food ingredient according to general process. Examples of the food comprises meat products, sausage, bread, chocolate, candy, snack, cracker, biscuit, pizza, ramen, noodle products, chewing gum, dairy products such as ice cream, soup, beverage, tea, drinks, alcohol drink, vitamin complex etc, but not intended herein to limit thereto, for preventing or improving of purposed disease.

The other components than aforementioned composition are various nutrients, a vitamin, a mineral or an electrolyte, synthetic flavoring agent, a coloring agent and improving agent in case of cheese, chocolate et al., pectic acid and the salt thereof, alginic acid and the salt thereof, organic acid, protective colloidal adhesive, pH controlling agent, stabilizer, a preservative, glycerin, alcohol, carbonizing agent used in carbonate beverage et al. The other component than aforementioned ones may be fruit juice for preparing natural fruit juice, fruit juice beverage and vegetable beverage, wherein the component can be used independently or in combination. The ratio of the components is not so important but is generally range from about 0 to 20 w/w % per 100 w/w% present composition.

Also, above described extract can be added to food or beverage for prevention and improvement of purposed disorder. The amount of above described extract in food or beverage as a functional health food or health supplement food may generally range from about 0.01 to 15 w/w% of total weight of food for functional health food composition. And the extract of the present invention may be added 0.02 to 5g, preferably 0.3 to 1g per 100ml in health beverage composition.

[Advantageous Effects]

As described in the present invention, inventive combined herb composition shows potent treating effect on human infertility, which is confirmed by various experiments, for example, *In vitro* sperm quality promoting effect evaluation as well as *in vivo* varicocele animal model evaluation method for investigating effects of the herbal extract on changes in sperm count and mobility in an *in vivo* varicocele animal model, it was verified that the inventive combined extract showed more potent increasing effect on the sperm count and sperm motility. Therefore, the herbal extract of the present invention can be usefully used in a pharmaceutical composition, health functional food, and health supplement food for preventing and treating male infertility.

[Description of Drawings]

[Best Mode]

The above and other objects, features and other advantages of the present invention will more clearly understood from the following detailed description taken in conjunction with the accompanying drawings, in which;

Fig. 1 shows the improving effect of test sample on the human seminal count and human seminal motility in human semen (the preliminary test on each test samples of CINTM1(10:1:10), CINTM1(5:1:5), and CINTM1(1:1:1) was performed before the test);

Fig. 2 shows the improving effect of test sample on the human seminal count and human seminal motility in human semen between extract of each herb and combined herb (CINTher-1:extract of *Morinda Officinalis root*; , CINTher-2:extract of *Cuscuta seed*; CINTher-3:extract of *Allium cepa bulb*; CINTM1; extract of combined herb mixed with 10:1:10, respectively);

Fig. 3 shows the improving effect of test sample on the sperm count in the varicocoele-induced animal group (CTR: Control, OP: Operation);

Fig. 4 presents the improving effect of test sample on the sperm motility in the varicocoele-induced animal group (CTR: Control, OP: Operation).

**Best Mode for Carrying Out the Invention**

It will be apparent to those skilled in the art that various modifications and variations can be made in the compositions, use and preparations of the present invention without departing from the spirit or scope of the invention.
The present invention is more specifically explained by the following examples. However, it should be understood that the present invention is not limited to these examples in any manner.

**EXAMPLES**

The following Examples and Experimental Examples are intended to further illustrate the present invention without limiting its scope.

**Example 1. Preparation of each component**

1-1. The extract of *Morinda officinalis root*

3,000g of dried root of *Morinda officinalis* purchased from Kyung-dong market (Dukhyundang Pharmacy) located in Seoul were washed and added to 6L of 95% ethanol to perform extraction with sonication extraction for 90mins 3 times. The residue was filtered with filter paper to afford extract and the filtrated extract was concentrated under vaccuo. The concentrated extract was dried with freeze dryer (ScanVac, Labogene) to afford 139.4g (Yield: 4.6%) of dried extract of *Morinda officinalis root* (designated as CINTher-1 hereinafter), which is used as a comparative test sample by dissolving into physiological saline solution and vigorously stirring for 2 mins to store at 4°C prior to use in following experiment.

1-2. The extract of *Cuscuta seed*

5.2kg of dried seed of *Cuscuta Chinensis* purchased from Kyung-dong market (Backjangsaeng Pharmacy) located in Seoul were washed, pulverized, and added to 5L of 95% ethanol to perform extraction with sonication extraction for 90mins 2 times. The residue was filtered with filter paper to afford extract and the filtrated extract was concentrated under vaccuo. The concentrated extract was dried with freeze dryer (ScanVac, Labogene) to afford 264.3g (Yield: 4.36%) of dried extract of *Cuscuta* seed (designated as CINTher-2 hereinafter), which is used as a comparative test sample by dissolving into physiological saline solution and vigorously stirring for 2 mins to store at 4°C prior to use in following experiment.

### 1-3. The extract of *Allium cepa bulb*

1,000g of bulb of *Allium cepa* purchased from Changnyeong market located in Kyungsangnamdo (Korea) were washed, dried and added to 5L of 70% ethanol to perform extraction with reflux extraction for 2 hours at 70°C. The residue was filtered with filter paper to afford extract and the filtrated extract was concentrated under vaccuo. The concentrated extract was dried with freeze dryer (ScanVac, Labogene) to afford 13.8g (Yield: 1.38%) of dried extract of *Allium cepa bulb* (designated as CINTher-3 hereinafter), which is used as a comparative test sample by dissolving into physiological saline solution and vigorously stirring for 2 mins to store at 4°C prior to use in following experiment.

### Example 2. Preparation of combined formulation (CINTM1)

The dried extract of *Morinda officinalis root, Cuscuta seed* and *Allium cepa bulb* prepared in Example 1 was thoroughly mixed with different mixed weigh ratios (10:1:10/ 5:1:5/ 1:1:1) using by mixer (Vortex genie-2, scientific industries, USA) to obtain the various kinds of invention formulation (designated as "CINTM1" hereinafter), which are used as a test samples in following experiment.

### Experimental Example 1. *In vitro* sperm quality promoting effect evaluation

In order to investigate the effect of the inventive extract obtained in Examples on the sperm number and sperm motility of human spermatozoa, following *in vitro* experiment was performed according the procedure disclosed in the literature (Martin Blomberg Jensen, Poul J.Bjerrum, Torben E.Jessen, John E. Nielsen, Ulla N.Joensen, et al. (2011) Vitamin D is positively associated with sperm motility and increases intracellular calcium in human spermatozoa. Human Reproduction, Vol.26, No.6 pp. 1307-1317).

### 1-1. Procedure

After determining the number of sperm and sperm motility of human semen delivered from called patients volunteers suffering from infertility (Urology division of Chonbuk National University Hospital in South Korea, 20 to 50 years old, 85 patients), the semen was poured into incubator maintaining 37°C. The liquefaction of the semen was sufficiently completed and then 200μL of test sample was added to 20μL of semen to the extent to reach 2mg/ml of final concentration. Three hour after the addition, the sperm motility was determined by using counting chamber (MAKLER counting chamber, self-medical instruments, Haifa, Israel) and microscope (Axiovert25, ZEISS, USA).

3H buffer solution {Hams F-10(N6635, Sigma chemical Co/ USA) + 0.4% HAS (A1635, Sigma Chemical Co. USA) + 12mM HERPES (H4034, Sigma Chemical Co. USA) [3H, pH 7.4]} was used as a control group. The test samples were also dissolved in the same buffer solution as mentioned the above, adjusted to pH 7.4 and centrifuged by centrifuge apparatus (A32010(1), LABOGENE, 13,000rpm, 2 mins, 4°C) to afford the supernatant for determination of sperm count and motility.

The sperm motility was calculated according to following empirical formulae 1.

[Empirical formulae 1]

$$\text{Percentage of motility} = A \text{ (the mean number of motile sperm)}/B \text{ (total number of sperms)}$$

### 1-2. Test Result

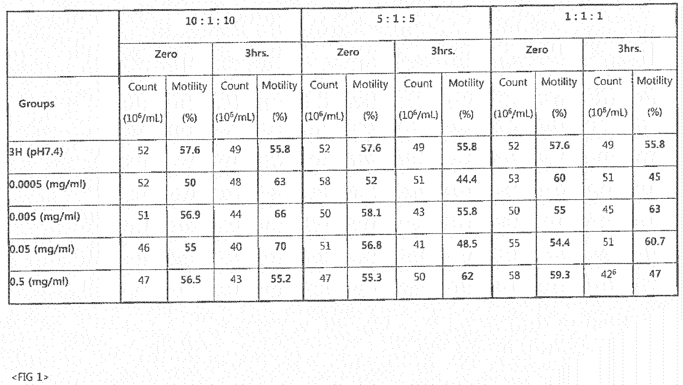

The sperm motility in the group treated with three kinds of inventive combinations, i.e., CINTM1(10:1:10), CINTM1(5:1:5), and CINTM1(1:1:1) was sharply increased by 70% in the group CINTM1(10:1:10) at the concentration of 0.05mg/ml, 62% in the group CINTM1(5:1:5) at the concentration of 0.5mg/ml and 63% in the group CINTM1(1:1:1) at the concentration of 0.5mg/ml, respectively. Among the test sample groups, the CINTM1 (10:1:10) group showed most potent increasing effect on sperm motility comparing with the other groups with different mixed ratio and the comparative test group treated with the extract of sole herb, i.e, CINTher-1, CINTher-2 and CINTher-3 (*See* Fig.1).

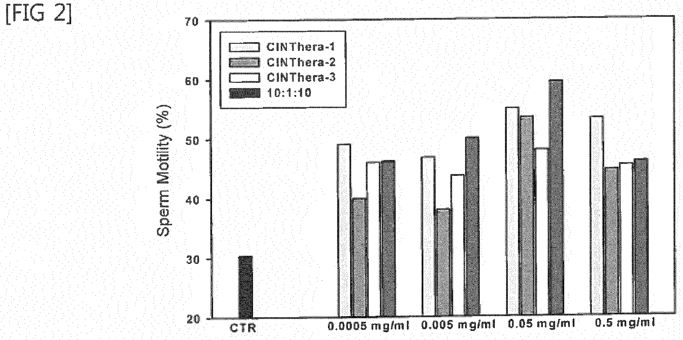

Specifically, the test groups treated with CINTM1(10:1:10) showed unexpectedly superior effect on the sperm motility at the same concentration (59.5%, 0.05mg/ml) comparing with the comparative test group treated with the extract of sole herb, i.e, CINTher-1(55%), CINTher-2 (53.5%) and CINTher-3(48.0%), respectively. (*See* Fig.2).

Accordingly, it has been confirmed that the combination of each herb (CINTM1) showed synergistic effect on sperm motility activity.

**Experimental Example 2. *In vivo* sperm quality promoting effect evaluation**

In order to investigate the effect of the inventive extract obtained in Examples on the sperm number and sperm motility of human spermatozoa, following *in vivo* experiment was performed according the procedure disclosed in the literature (Zhang LT, Kim HK, Choi BR, Zhao C, Lee SW, Jang KY, et al.(2014) Analysis of testicular-internal spermatic vein variation and the recreation of varicocoele in a Sprague-Dawley rat model. Andrology. 2, 466-73).

2-1. Establishment of *In vivo* varicocoele animal model

In order to establish *In vivo* varicocoele animal model for use in following experiment, 9 weeks old Sprague-Dawley rats weighing 300 to 350g (purchased from KOATECH Co. Ltd located at Pyeongtaek city, KOREA) were bred after acclimating to environment for 1 week. The rats divided with four rats per cage, were fed standard rat chow and freely access to purified water. The rats were maintained in the animal facility under constantly controlled environmental conditions (internal temperature: 18 to 23°C, relative humidity: 40 to 60%) and 12 hours light-dark cycle).

Each rat was intraperitoneally anaesthetized using by a mixture of Ketamine (50mg/kg) and Xylazine (25mg/kg) and the renal vein connected to venae cava was exposed after approaching left renal through a midline abdominal incision. The left renal vein was ligated with a 5.0 nylon suture with a metal probe (outer diameter: 0.85mm) and the metal probe was withdrawn, which leads to 50% reduction in the diameter of the left renal vein and a consistent constriction of the vessel at the point of the ligation. ISV (Internal spermatic vein) branch was ligated to induce varicocoele for use as a varicocoele-induced animal group and four weeks after the ligation, the test samples dissolved in physiological saline solution were orally administrated into the experimental rats.

2-2. Evaluation of potency in *In vivo* animal model

2ml/day of test samples or physiological saline solution (PSA) had been orally administrated into two test groups for 28 days after the operation, i.e., varicocoele-induced animal group {(1) PSA: 2ml/day, (2) CINTM1(10:1:10): 100mg/kg/2ml/day, (3) CINTM1(10:1:10):200mg/kg/2ml/day} and normal control group {(1) PSA: 2ml/day, (2) CINTM1(10:1:10): 100mg/kg/2ml/day, (3) CINTM1(10:1:10): 200mg/kg/2ml/day} and then the weight of rats was weighed. The seminal duct of rat was delivered under general anesthesia. The sperm count and motility in the seminal duct were determined and the weight of bilateral testis and epididymis was weighed to compare.

2-3. Test Result

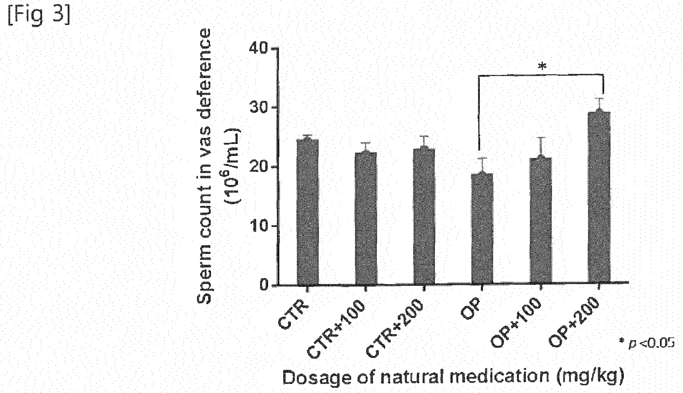

As shown in Fig. 3, the sperm count in the varicocoele-induced animal group treated with inventive combination showed sharply increased comparing with the group treated with PSA. The sperm count in the varicocoele-induced animal group treated with inventive combination was $20.91 \pm 12.25 \times 10^6$/ml (100mg/kg/2ml/day) and $28.63 \pm 8.90 \times 10^6$/ml (200mg/kg/2ml/day) while that in the varicocoele-induced animal group treated with PSA was only $18.25 \pm 9.53 \times 10^6$/ml, (*See* Fig.3).

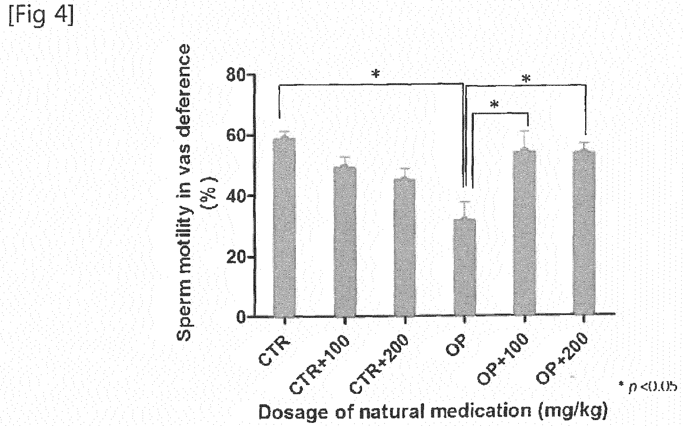

As shown in Fig. 4, the sperm motility in the varicocoele-induced animal group treated with inventive combination showed sharply increased comparing with the group treated with PSA. The sperm motility in the varicocoele-induced animal group treated with inventive combination was $53.8 \pm 20.7\%$ (100mg/kg/2ml/day) and $53.3 \pm 12.1\%$ (200mg/kg/2ml/day) while that in the varicocoele-induced animal group treated with PSA was only $31.3 \pm 18.8\%$. (*See* Fig.4).

[Mode for Invention]

Hereinafter, the formulating methods and kinds of excipients will be described, but the present invention is not limited to them. The representative preparation examples were described as follows.

1. Preparation of powder

| | |
|---|---|
| CINTM1 | 20mg |
| Lactose | 100mg |
| Talc | 10mg |

Powder preparation was prepared by mixing above components and filling sealed package.

2. Preparation of tablet

| | |
|---|---|
| CINTM1 | 10mg |
| Corn Starch | 100mg |
| Lactose | 100mg |
| Magnesium stearate | 2mg |

Tablet preparation was prepared by mixing above components and entabletting.

3. Preparation of capsule

| | |
|---|---|
| CINTM1 | 10mg |
| Crystalline cellulose | 3mg |
| Lactose | 14.8mg |
| Magnesium stearate | 0.2mg |

Capsule preparation was prepared by mixing above components and filling gelatin capsule by conventional gelatin preparation method.

4. Preparation of injection

| | |
|---|---|
| CINTM1 | 10mg |
| Mannitol | 180mg |
| Sterilized distilled water for injection | 2974mg |
| $Na_2HPO_4 \cdot 12H_2O$ | 26mg |

Injection preparation was prepared by dissolving active component, and then filling all the components in $2m\ell$ per ample by conventional injection preparation method.

5. Preparation of liquid

| | |
|---|---|
| CINTM1 | 20mg |
| Isomerized glucose | 10g |
| Mannitol | 5g |
| distilled water | optimum amount |

Liquid preparation was prepared by dissolving active component in distilled water, adding lemon flavor, mixing together with distilled water and then filling all the components in $100m\ell$ brown bottle and sterilizing by conventional liquid preparation method.

6. Preparation of health food

| | |
|---|---|
| CINTM1 | 1000mg |
| Vitamin mixture | optimum amount |
| Vitamin A acetate | 70$\mu$g |
| Vitamin E | 1.0mg |
| Vitamin $B_1$ | 0.13mg |
| Vitamin $B_2$ | 0.15mg |
| Vitamin $B_6$ | 0.5mg |
| Vitamin $B_{12}$ | 0.2$\mu$g |
| Vitamin C | 10mg |
| Biotin | 10$\mu$g |
| Amide nicotinic acid | 1.7mg |
| Folic acid | 50$\mu$g |
| Calcium pantothenic acid | 0.5mg |
| Mineral mixture | optimum amount |
| Ferrous sulfate | 1.75mg |
| Zinc oxide | 0.82mg |
| Magnesium carbonate | 25.3mg |
| Monopotassium phosphate | 15mg |
| Dicalcium phosphate | 55mg |
| Potassium citrate | 90mg |
| Calcium carbonate | 100mg |
| Magnesium chloride | 24.8mg |

The above mentioned vitamin and mineral mixture may be varied in many ways. Such variations are not to be regarded as a departure from the spirit and scope of the present invention.

7. Preparation of health beverage

| | |
|---|---|
| CINTM1 | 1000mg |
| Citric acid | 1000mg |
| Oligosaccharide | 100g |
| Apricot concentration | 2g |
| Taurine | 1g |
| Distilled water | 900m$\ell$ |

Health beverage preparation was prepared by dissolving active component, mixing, stirred at 85 °C for 1 hour, filtered and then filling all the components in 2L ample and sterilizing by conventional health beverage preparation method.
The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

[Industrial Applicability]

As described in the present invention, the combined herb composition shows potent treating effect on human infertility, which is confirmed by various experiments, for example, *In vitro* sperm quality promoting effect evaluation as well as *in vivo* varicocele animal model evaluation method for investigating effects of the herbal extract on changes in sperm count and mobility in an in vivo varicocele animal model, it was verified that the inventive combined extract showed more potent increasing effect on the sperm count and sperm motility. Therefore, the herbal extract of the present invention can be usefully used in a pharmaceutical composition, health functional food, and health supplement food for preventing and treating male infertility.

**Claims**

1. A pharmaceutical composition comprising the combined herb extract of *Morinda Officinalis root, Cuscuta seed,* and *Allium cepa bulb* as an active ingredient for preventing and treating male infertility disease.

2. The pharmaceutical composition according to claim 1, wherein said pharmaceutical composition comprises the combined herb extract of *Morinda Officinalis root, Cuscuta seed,* and *Allium cepa bulb* with the mixed ratio based on the dried weight of each herb (w/w) of 0.01 - 100: 0.01-1: 1-100(w/w).

3. The pharmaceutical composition according to claim 1, wherein said male infertility disease is selected from a male infertility disease caused by the adverse response of anti-cancer agent, azoospermia, oligospermia, asthenospermia, or teratospermia.

4. A health functional food comprising the combined herb extract of *Morinda Officinalis root*, *Cuscuta seed*, and *Allium cepa bulb* as an active ingredient for the prevention or improvement of male infertility disease.

5. The health functional food according to claim 4, wherein said health functional food is provided as powder, granule, tablet, capsule, pill, suspension, emulsion, syrup, tea bag, leached tea, or beverage type.

6. A health supplement food comprising the combined herb extract of *Morinda Officinalis root, Cuscuta seed,* and *Allium cepa bulb* as a main component, for the prevention or improvement of male infertility disease.

7. A method of treatment comprising administering of the composition comprising the combined herb extract of *Morinda Officinalis root, Cuscuta seed,* and *Allium cepa bulb* to a subject in need of treatment of male infertility diseases.

8. A use of the combined herb extract of *Morinda Officinalis root, Cuscuta seed,* and *Allium cepa bulb* for manufacture of medicament employed for treating male infertility disease in human or mammal.

| Groups | 10 : 1 : 10 | | | | 5 : 1 : 5 | | | | 1 : 1 : 1 | | | |
| | Zero | | 3hrs. | | Zero | | 3hrs. | | Zero | | 3hrs. | |
| | Count $(10^6/mL)$ | Motility (%) | Count $(10^6/mL)$ | Motility (%) | Count $(10^6/mL)$ | Motility (%) | Count $(10^6/mL)$ | Motility (%) | Count $(10^6/mL)$ | Motility (%) | Count $(10^6/mL)$ | Motility (%) |
|---|---|---|---|---|---|---|---|---|---|---|---|---|
| 3H (pH7.4) | 52 | 57.6 | 49 | 55.8 | 52 | 57.6 | 49 | 55.8 | 52 | 57.6 | 49 | 55.8 |
| 0.0005 (mg/ml) | 52 | 50 | 48 | 63 | 58 | 52 | 51 | 44.4 | 53 | 60 | 51 | 45 |
| 0.005 (mg/ml) | 51 | 56.9 | 44 | 66 | 50 | 58.1 | 43 | 55.8 | 50 | 55 | 45 | 63 |
| 0.05 (mg/ml) | 46 | 55 | 40 | 70 | 51 | 56.8 | 41 | 48.5 | 55 | 54.4 | 51 | 60.7 |
| 0.5 (mg/ml) | 47 | 56.5 | 43 | 55.2 | 47 | 55.3 | 50 | 62 | 58 | 59.3 | $42^6$ | 47 |

<FIG 1>

[FIG 2]

[Fig 3]

[Fig 4]

## INTERNATIONAL SEARCH REPORT

| International application No. |
|---|
| **PCT/KR2015/003506** |

**A. CLASSIFICATION OF SUBJECT MATTER**

*A61K 36/74(2006.01)i, A61K 36/43(2006.01)i, A61K 36/8962(2006.01)i, A61P 15/00(2006.01)i*

According to International Patent Classification (IPC) or to both national classification and IPC

**B. FIELDS SEARCHED**

Minimum documentation searched (classification system followed by classification symbols)
A61K 36/74; A61K 36/40; A61K 31/352; A61K 31/7048; A61K 36/73; A61K 36/43; A61K 36/8962; A61P 15/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: Monrinda officinalis, dodder, Allium cepa, male infertility

**C. DOCUMENTS CONSIDERED TO BE RELEVANT**

| Category* | Citation of document, with indication, where appropriate, of the relevant passages | Relevant to claim No. |
|---|---|---|
| Y | OH, Jae - Sung et al.. "Administration Duration Dependent Effects of Morindae Radix Extract Solution on Reproductive Capacities in Mice." Journal of The Korea Institute of Oriental Medical Diagnostics. 2006. vol. 27, no. 3, pages 63-76 See abstract, page 74. | 1-6,8 |
| Y | PARK, Jeong Yeol. "Effects of Cuscutae Semen on the cAMP-Responsive Element Modulator (CREM) Expression and Sperm Parameters in Infertile Male Mice Induced by Cyclophosphamide." Doctoral Thesis of Department of Korean Medicine of Graduate School, Kyung Hee University. February 2011 See abstract in Korean. | 1-6,8 |
| Y | KHAKI, A. et al.. "Evaluation of androgenic activity of allium cepa on spermatogenesis in the rat." Folia Morphol (Warsz). 2009. Vol. 68, No. 1, Abstract pp. 45-51 See abstract. | 1-6,8 |
| A | KR 10-2013-0035091 A (THE CATHOLIC UNIVERSITY OF KOREA INDUSTRY-ACADEMIC COOPERATION FOUNDATION) 08 April 2013 See the entire document. | 1-6,8 |
| A | KR 10-2007-0094394 A (SEOUL PERFUMERY et al.) 20 September 2007 See the entire document. | 1-6,8 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 JUNE 2015 (25.06.2015) | **26 JUNE 2015 (26.06.2015)** |

| Name and mailing address of the ISA/**KR** | Authorized officer |
|---|---|
| Korean Intellectual Property Office Government Complex-Daejeon, 189 Seonsa-ro, Daejeon 302-701, Republic of Korea Facsimile No. 82-42-472-7140 | Telephone No. |

Form PCT/ISA/210 (second sheet) (July 2009)

## INTERNATIONAL SEARCH REPORT

International application No.

**PCT/KR2015/003506**

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 7
   because they relate to subject matter not required to be searched by this Authority, namely:

   Claim 7 pertains to a method for treatment of the human body by surgery or therapy, and to a diagnostic method, and thus it is considered that claim 7 pertains to subject matter on which the International Searching Authority is not required to carry out an international search [PCT Article 17(2)(a)(i) and PCT Rule 39.1(iv)].

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT
Information on patent family members

International application No.

**PCT/KR2015/003506**

| Patent document cited in search report | Publication date | Patent family member | Publication date |
|---|---|---|---|
| KR 10-2013-0035091 A | 08/04/2013 | KR 10-1341648 B1<br>US 2013-0085114 A1<br>US 8980847 B2 | 16/12/2013<br>04/04/2013<br>17/03/2015 |
| KR 10-2007-0094394 A | 20/09/2007 | KR 10-0803289 B1 | 13/02/2008 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **AGARWAL A ; DEPPINDER F ; COCUZZA M ; AGARWAL R ; SHORT RA ; SABANEGH E. et al.** fficacy of varicocelectomy in improving semen parameters: new meta-analytical approach. *Urology,* 2007, vol. 70, 532-538
- **AGARWAL A ; SHARMA RK ; DESAI NR ; PRABAKARAN S ; TABANEGH E.** Role of Oxidative Stress is Pathogenesis of Varicocele and Infertility. *Urology,* 2009, vol. 73, 461-469
- **MEHTA A ; GOLDSTEIN M.** Microsurgical varicocelectomy: a review. *Asian J. Androl.,* 2012, vol. 15, 56-60
- **CHENG D ; ZHENG XM ; LI SW ; YANG ZW ; HU LQ.** Effects of epidermal growth factor on sperm content and motility of rats with surgically induced varicoceles. *Asian J Androl,* 2006, vol. 8, 713-717
- **DING H ; TIAN JQ ; DU W ; ZHANG LY ; WANG HZ ; WANG ZP.** Open non-microsurgical, laparoscopic or open microsurgical varicocelectomy for male infertility: a meta-analysis of randomized controlled trials. *BJU Int,* 2012, vol. 110, 1536-1542
- **FISCH H ; HYUN G.** Varicocele repair for low testosterone. *Curr Opin Urol,* 2012, vol. 22, 495-498
- **FRETZ PC ; SANDLOW JI.** Varicocele: current concepts in pathophysiology, diagnosis, and treatment. *Urol Clin North Am,* 2002, vol. 29, 921-937
- **MEHTA A ; GOLDSTEIN M.** Microsurgical varicocelectomy: a review. *Asian J Androl,* 2013, vol. 15, 56-60
- **TANRIVERDI O ; MIROGLU C ; HORASANLI K ; ALTAY B ; CALISKAN KC ; GUMUS E.** Testicular blood flow measurements and mean resistive index values after microsurgical and high ligation varicocelectomy. *Urology,* 2006, vol. 67, 1262-1265
- **EVERS JL ; COLLINS JA.** Assessment of efficacy of varicocele repair for male subfertility: a systematic review. *The Lancet,* 2003, vol. 361, 1849-1852
- **FICARRA V ; CERRUTO MA ; LIGUORI G ; MAZZONI G ; MINUCCI S ; TRACIA A et al.** Treatment of varicocele in subfertile men: the Cochrane review a contrary opinion. *Eur Urol,* 2006, vol. 49, 258-263
- **AGARWAL A ; SHARMA RK ; DESAI NR ; PRABAKARAN S ; TAVARES A ; SABANEGH E.** Role of Oxidative Stress in Pathogenesis of Varicocele and Infertility. *Urology,* 2009, vol. 73, 461-469
- **SHARMA R ; AGARWAL A ; MOHANTY G ; HAMADA AJ ; GOPALAN B ; WILLARD B et al.** Proteomic analysis of human spermatozoa proteins with oxidative stress. *Reprod Biol Endocrinol,* 2013, vol. 11, 48
- **MARK SIGMAN ; STACY GLASS ; JANICE CAMPAGNONE ; JOHN L. REYOR.** Carnitine for the treatment of idiopathic asthenospermia: a randomized. double-blind, placebo-controlled trial. *Fertility and Sterility,* 2006, vol. 85 (5
- **B. S. CHUNG et al.** YoungRim Press. DohaeHyangYakDaeSaJeon, 1998, 927-928
- **B. S. CHUNG et al.** YoungRim Press. DohaeHyangYak DaeSaJeon, 1998, 882-883
- **B. S. CHUNG et al.** YoungRim Press. DohaeHyangYakDaeSaJeon, 1998, 155-156
- Textbook of andrology. Korean Society for sexual medicine and andrology, 2010
- **MARTIN BLOMBERG JENSEN ; POUL J.BJERRUM ; TORBEN E.JESSEN ; JOHN E. NIELSEN ; ULLA N.JOENSEN et al.** Vitamin D is positively associated with sperm motility and increases intracellular calcium in human spermatozoa. *Human Reproduction,* 2011, vol. 26 (6), 1307-1317
- **ZHANG LT ; KIM HK ; CHOI BR ; ZHAO C ; LEE SW ; JANG KY et al.** Analysis of testicular-internal spermatic vein variation and the recreation of varicocele in a Sprague-Dawley rat model. *Andrology,* 2014, vol. 2, 466-73